(19) Europäisches Patentamt / European Patent Office / Office européen des brevets

(11) EP 3 600 462 B1

# (12) EUROPEAN PATENT SPECIFICATION

(45) Date of publication and mention of the grant of the patent:
**21.09.2022 Bulletin 2022/38**

(21) Application number: **18777120.9**

(22) Date of filing: **02.04.2018**

(51) International Patent Classification (IPC):
***A61L 26/00*** *(2006.01)*

(52) Cooperative Patent Classification (CPC):
(C-Sets available)
**A61L 26/0085; A61L 26/0009; A61L 26/0023;**
A61L 2400/04 (Cont.)

(86) International application number:
**PCT/US2018/025742**

(87) International publication number:
**WO 2018/184021 (04.10.2018 Gazette 2018/40)**

## (54) HYDROPHOBICALLY-MODIFIED POLYMER FOAMS AND METHODS OF USE

HYDROPHOB MODIFIZIERTE POLYMERSCHAUMSTOFFE UND VERFAHREN ZUR VERWENDUNG

MOUSSES POLYMÈRES MODIFIÉES HYDROPHOBIQUEMENT ET PROCÉDÉS D'UTILISATION

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **31.03.2017 US 201762479903 P**

(43) Date of publication of application:
**05.02.2020 Bulletin 2020/06**

(73) Proprietor: **Medcura, Inc.**
**Riverdale, Maryland 20737 (US)**

(72) Inventor: **DOWLING, Matthew**
**Riverdale, MD 20737 (US)**

(74) Representative: **Grund, Martin**
**Grund Intellectual Property Group**
**Patentanwälte und Solicitor PartG mbB**
**Steinsdorfstraße 2**
**80538 München (DE)**

(56) References cited:
**US-A1- 2002 042 473**
**US-A1- 2005 226 916**
**US-A1- 2008 213 243**
**US-A1- 2012 123 356**
**US-A1- 2012 265 287**
**US-A1- 2014 336 147**
**US-A1- 2016 206 777**

- **DOWLING et al.: "A self-assembling hydrophobically modified chitosan capable of reversible hemostatic action", Biomaterials, vol. 32, no. 13, 22 December 2010 (2010-12-22), pages 3351-3357, XP028366478,**
- **LIU et al.: "Smart gelation of chitosan solution in the presence of NaHC03 for injectable drug delivery system", International Journal of Pharmaceutics, vol. 414, no. 1-2, 19 April 2011 (2011-04-19), pages 6-t5, XP028233919,**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

(52) Cooperative Patent Classification (CPC): (Cont.)

C-Sets
**A61L 26/0009, C08L 101/02;**
**A61L 26/0023, C08L 5/08**

## Description

This application claims the benefit of U.S. Provisional Application No. 62/479,903, filed March 31, 2017.

## BACKGROUND

Bleeds can range from small, superficial bleeds to those caused by traumatic injuries, hematological disorders, and invasive medical procedures, among others. When the site of bleeding is not readily accessible, such as inside a body cavity, hemorrhage control within the body cavity is difficult to achieve and can lead to many complications. For example, uncontrolled bleeding remains the leading cause of death in the battlefield.

Accordingly, this is a need for compositions and methods that provide effective hemostatic control, including for bleeds that are not easily accessible with hemostatic devices. The present invention addresses these needs and other objectives.

US 2016/206777 A1 discloses a hybrid composition of matter that provides a strongly adhesive hemostatic agent. The composition of matter can be formulated in a liquid state as a spray solution. The solution is composed, at least in part, of a hydrophobically modified biopolymer (e.g. chitosan) matrix capable of hydrophobically interacting with and/or gelling with red blood cells. A system for delivering the solution is also disclosed including two or more containers operationally connected through an ejection mechanism for ejecting a mixture of the solution and various other secondary components.

## SUMMARY

In various aspects and embodiments, the present invention provides hemostat products and methods of treating bleeds, including cavity bleeds or other difficult to access bleeds, including nose bleeds (epistaxis).

In various embodiments, the invention provides a device having at least two compartments each containing a releasable, flowable product. The first compartment comprises a solution of an acidified hydrophobically-modified chitosan that gels or self-assembles in the presence of blood. The hydrophobically-modified chitosan includes hydrophobic grafts larger than C12, and from 5% to about 40% of the hydrophobic grafts are C16 or C18. A second compartment comprises a solution of a bicarbonate or carbonate salt at an alkaline pH. Delivery of the contents to a bleed results in the production of a stable foam that can quickly control bleeds, including bleeds that are difficult to access, and/or that are not easily amenable to compression.

In some embodiments, the container is any container that allows for controlled release of the contents, such as a syringe. In some embodiments, the product comprises a double-barrel syringe comprising two compartments and a static mixing tip. The static mixing tip stabilizes carbon dioxide ($CO_2$) gas and allows for direct application of a hydrophobically modified-polymer foam.

The first compartment of the container contains a modified polymer, which is a hydrophobically modified chitosan. In some embodiments, the modified polymer is modified with C8 to C20 hydrophobic grafts (which can be linear hydrocarbon grafts present along the backbone of the polymer. In the case of hydrophobically-modified chitosan, about 10% to about 50% of available functional groups along the backbone may be occupied by the hydrophobic grafts. In some embodiments, the hm-chitosan includes C12 grafts as well as some grafts larger than C12 to provide added integrity to the foam. In some embodiments, from 5% to about 40% of the grafts are C16 or C18. In some embodiments, the modified polymer (e.g., hm-chitosan) may be present in the first container at about 0.1 to about 2% by weight of the solution in the first compartment, such as from 0.2 to 0.8% by weight in some embodiments. The modified polymer may be formulated at a pH of from 2.5 to 4.5, and in some embodiments from pH 3 to pH 4.5. In some embodiments, the modified polymer is dissolved in acetic acid, lactic acid, ascorbic acid, or citric acid. For example, in the case of acetic acid and hm-chitosan, the acetic acid may be from 0.3 to 0.5M.

The second compartment of the container comprises a solution of a carbonate or bicarbonate salt, such as sodium bicarbonate. In some embodiments, the bicarbonate solution is from 0.2 to 0.5M. The bicarbonate or carbonate solution may be at pH 8 to 10, or from about pH 8 to about pH 9. In some embodiments, second compartment further comprises one or more negatively charged polymers, which in some embodiments are hydrophobically modified (e.g., hydrophobically modified alginate).

Upon mixing of the first compartment and the second compartment using the mixing tip, an expanding foam is generated due to stabilization of bubbles formed from the generation of $CO_2$ gas. In various embodiments, the foam is stable for at least 10 minutes, is stable when mixed with blood, and allows for transfer of gases in some embodiments.

As disclosed herein, a product of the present disclosure is for use in methods related to treating bleeds, including cavity bleeds (e.g., epistaxis), by releasing the contents of the product onto the site of bleeding. For example, the bleeding wound may be epistaxis and the contents of the container may be released into one or more nostrils. In some embodiments, the resulting foam or gel is applied to the naval cavity through a conical applicator, or is attached to a substrate

that is removed from the nostril once the foam/gel is applied.

Other aspects and embodiments of the invention will be apparent from the following detailed description.

DESCRIPTION OF THE FIGURES

Figure 1A illustrates a dual barrel syringe for creating Hm-chitosan foam. Hm-chitosan is solubilized in acetic acid in Side A. Upon delivery, HmChitosan will be mixed with sodium bicarbonate from Side B.

Figure 1B illustrates production of a stable foam upon generation of $CO_2$ gas.

Figure 2 is a graph showing surface tension measurements for Hm-chitosan with varying modification degree with C12 hydrophobic grafts.

Figure 3 is a graph showing a representative frequency sweep for Hm-chitosan foam and solution. Circles represent 0.3wt% Hm-chitosan hybrid in 0.04M AcOH/0.15M NaAcetate. Squares represent a pure solution of the same formulation. Final concentrations are reported.

Figure 4 is a graph showing storage modulus for foam mixtures. Foam and blood (or PBS) were mixed at a 1:1 ratio by weight. Pre-mix foam formulation was 0.3wt% Hm-chitosan hybrid in 0.04M AcOH/0.15M NaAcetate. Storage modulus (and loss modulus, not shown) are preserved when mixing foam with blood. Significant decrease in moduli observed when mixing with PBS (foam is obliterated).

Figure 5 is a graph showing a time sweep at 1Hz for HmChitosan foam. Foam formulation: 0.3wt% Hm-chitosan hybrid in 0.040M AcOH/0.15M NaAcetate.

Figure 6 is a graph showing a representative strain sweep for 0.3wt% Hm-chitosan hybrid in 0.04M AcOH/0.15M NaAcetate foam.

Figure 7 illustrates the overall experimental set up for the *in vivo* rat anterior epistaxis model

Figure 8 is an image showing the saline/no treatment control for the experiment.

Figure 9 is an image showing the HM-chitosan gauze packing treatment for the experiment.

Figure 10 is a graph showing the results of average blood loss in the experiment for different treatments, including the control gauze and the no treatment control.

Figure 11 is a graph showing average cumulative blood loss for different treatments and controls.

Figure 12 is a line graph showing the results of blood loss per minute post nasal punch.

Figure 13 is a graph showing the results of the time between the first and last blood droplet.

DETAILED DESCRIPTION

In various aspects and embodiments, the present invention provides hemostat products and methods of treating bleeds, including cavity bleeds or other difficult to access bleeds, including nose bleeds (epistaxis). In various embodiments, the invention provides a device having at least two compartments each containing a releasable, flowable product. The first compartment comprises a solution of an acidified hydrophobically-modified chitosan that gels or self-assembles in the presence of blood. The hydrophobically-modified chitosan includes hydrophobic grafts larger than C12, and from 5% to about 40% of the hydrophobic grafts are C16 or C18. A second compartment comprises a solution of a bicarbonate or carbonate salt at an alkaline pH. Delivery of the contents to a bleed results in the production of a stable foam that can quickly control bleeds, including bleeds that are difficult to access, and/or that are not easily amenable to control by compression. In accordance with embodiments of the invention, the foams are stable for at least 10 minutes (e.g., do not immediately dissipate) and remain stable in the presence of blood.

In some embodiments, the container is any container that allows for controlled release of the contents, such as a syringe. The ratio of volume released in various embodiments can be from 1:0.5 to 0.5:1 of the first and second containers. In some embodiments, the ratio of the solution in the first compartment and the ratio of the solution in the second compartment are about 1:1. In some embodiments, the product comprises a double-barrel syringe comprising two compartments and a static mixing tip. The static mixing tip stabilizes carbon dioxide ($CO_2$) gas and allows for direct application of a hydrophobically modified foam.

The first compartment of the container contains a modified polymer, which is hydrophobically-modified chitosan. The modified polymer (i.e. hm-chitosan) may be present in the first container at 0.1 to about 2% by weight, or about 0.1 to about 1% by weight, or about 0.2 to about 0.8% by weight (e.g., about 0.5-0.6% by weight) of the solution in the first compartment. The modified polymer may be formulated at a pH of from 2.5 to 4.5, and in some embodiments is preferably in the range of pH 3 to pH 4.5. In some embodiments, the modified polymer is dissolved in acetic acid, lactic acid, ascorbic acid, or citric acid. For example, in the case of acetic acid and hm-chitosan, the acetic acid may be from 0.3 to 0.5M, or in some embodiments, from 0.35 to 0.45 M. In some embodiments, lactic acid is used to avoid offensive odor.

The second compartment of the container comprises a solution of a carbonate or bicarbonate salt, such as

sodium bicarbonate. In the second compartment, the bicarbonate solution may be around 0.1 wt% to about 1 wt%, or from about 0.2 wt% to about 0.5 wt%. In some embodiments, the bicarbonate solution is from 0.2 to 0.5M, such as from about 0.2 to about 0.4 M, or about 0.3M. The bicarbonate or carbonate solution may be at pH 8 to pH 10, or from about pH 8 to about pH 9. In some embodiments, the modified polymer is placed in the second compartment. For example, hydrophobically modified alginate can be placed in the second compartment. In other embodiments employing hm-chitosan in the first compartment, the second compartment may further comprise a negatively charged polymer, which may improve the integrity of the resulting foam. Exemplary negative charged polymers include alginate, pectin, dextran, carboxymethylcellulose, or xanthan gum. In some embodiments, the negatively charged polymer is hydrophobically modified (e.g., hydrophobically-modified alginate).

Upon mixing of the first compartment and the second compartment using the mixing tip, an expanding foam is generated due to stabilization of bubbles formed from the generation of $CO_2$ gas. In general, a smaller bubble size yields a longer drainage time, and therefore, longer stability. Foam stability (i.e., 'foamability') can be adjusted based on hydrophobic modification degrees and types, and also on the hydrophobically modified polymer's molecular weight. In addition, the static mixer foams of the present disclosure are able to form in situ, such that foam may be generated within the blood. As demonstrated in the experiments herein, the storage modulus for the foam mixtures is preserved when mixed with blood but not with PBS. This may be a demonstration of the interaction between blood cells and the modified polymer, such as gelation or aggregation.

As disclosed herein, a product of the present disclosure may be for use in methods related to treating a bleeds, including cavity bleeds (e.g., epistaxis), by releasing the contents of the product onto the site of bleeding. For example, the bleeding wound may be epistaxis and the contents of the container may be released into one or more nostrils. In some embodiments, the resulting foam or a gel is applied to the naval cavity through a conical applicator, or is attached to a substrate that is removed from the nostril once the foam/gel is applied.

The hm-polymer material for use in accordance with embodiments of the invention is chitosan. Chitosan is the common name of the linear, random copolymer that consists of β-(1-4)-linked D-glucosamine and N-acetyl-D-glucosamine. The molecular structure of chitosan consists of a linear backbone linked with glycosidic bonds. Chitosan is the major component of crustacean shells such as crab, shrimp, krill and crawfish shells. Additionally, chitosan is the second most abundant natural biopolymer after cellulose. Commercial chitosan samples are typically prepared by chemical de-N-acetylation of chitin under alkaline conditions. Depending on the source of the natural chitin (extracted from shells) and its production process, chitosan can differ in size (average molecular weight Mw) and degree of N-acetylation (%DA). While the poor solubility of chitosan in water and in common organic solvents restricts its applications, reactive amino groups in the chitosan backbone make it possible to chemically conjugate chitosan with various molecules and to modulate its properties for use as a hemostat product.

In various embodiments, the degree of deacetylation of chitin may range from about 40-100%, or in some embodiments, from 60 to 100%, or from 40 to about 90%, or from 50 to about 80%, which determines the charge density. The structure of chitosan (deacetylated) is depicted in Formula 1:

Formula 1

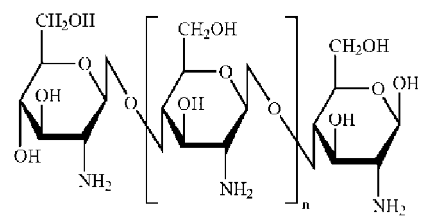

These repeating monomeric units include a free amino group, which makes molecules or compounds containing chitosan or its derivatives readily reactive. The hydrophobic modification of the chitosan backbone is through the association of an amphiphilic compound with the amino group, such that the hydrophobic tail of the amphiphilic compound is bound with the hydrophilic backbone structure.

The polymer that forms the backbone is chitosan, or similar polymer of synthetic or natural origin, including for example, water-soluble polysaccharides and water-soluble polypeptides. In some embodiments, the polymer is one or more hm-polysaccharides, including but not limited to cellulosics, chitosans and alginates, all of which are abundant, natural biopolymers. In some embodiments, the hm-biopolymer contains cationic groups.

The natural origin of these polysaccharides varies, cellulosics are found in plants, whereas chitosans and alginates are found in the exoskeleton or outer membrane of a variety of living organisms. Many of these naturally

occurring polymers, in addition to being able to form long stable chains for forming the polymer backbone, have properties that are beneficial for a hemostat application, including anti-microbial properties.

In some embodiments, the hm-chitosan is derived from a deacteylated chitin, which may be derived from one or more of crab, shrimp, krill, and crawfish.

The form of the natural polymers used may vary to include standard states, derivatives and other various formulations. For example, the hm-cellulosics may be formed from, without limitation, hydroxyethyl cellulose, hydroxypropyl cellulose, methyl cellulose, hydroxypropyl methyl cellulose, and/or hydroethyl methyl cellulose. Hm-chitosans may be prepared from, without limitation, the following chitosan salts: chitosan lactate, chitosan salicylate, chitosan pyrrolidone carboxylate, chitosan itaconate, chitosan niacinate, chitosan formate, chitosan acetate, chitosan gallate, chitosan glutamate, chitosan maleate, chitosan aspartate, chitosan glycolate and quaternary amine substituted chitosan and salts thereof. Hm-alginates may be prepared from, without limitation, sodium alginate, potassium alginate, magnesium alginate, calcium alginate, and/or aluminum alginate.

In some embodiments, the polymeric component is a mixture of polysaccharides. For instance, the mixture may be of various different sub-classes of a single polymer class. Alternatively, the mixture may include two or more different classes of polymer, for instance a chitosan and an alginate.

In various embodiments, the biopolymer is an hm-chitosan, which may be prepared from a chitosan having a degree of deacetylation of from about 40 to about 90%, or from about 50 to about 80%, or from about 60% to about 75%. The polymer may have 10% to about 50% of functional groups occupied by a hydrophobic group. In some embodiments, the degree of substitution of the hydrophobic substituent on the biopolymer is from about 1 to about 100 moles of the hydrophobic group per mole of the polymer. In some embodiments, the degree of substitution of the hydrophobic substituent on the biopolymer is from about 5 to about 100 moles of the hydrophobic group per mole of the polymer. In some embodiments, the degree of substitution of the hydrophobic substituent on the biopolymer is from about 5 to about 50 moles of the hydrophobic group per mole of the polymer. In some embodiments, the degree of substitution of the hydrophobic substituent on the biopolymer is from about 10 to about 50 moles of the hydrophobic group per mole of the polymer. In some embodiments, the degree of substitution of the hydrophobic substituent on the biopolymer is from about 10 to about 30 moles of the hydrophobic group per mole of the polymer. In some embodiments, the degree of substitution of the hydrophobic substituent on the polysaccharide is from about 40 to 65 moles of the hydrophobic substituent per mole of the polysaccharide. In some embodiments, the degree of substitution of the hydrophobic substituent on the polysaccharide is from about 1 to 30 moles of the hydrophobic substituent per mole of the polysaccharide. In some embodiments, the molecular weight of the polysaccharides used as the biopolymer range from about 25,000 to about 1,500,000 grams per mole. In various embodiments, the molecular weight of the biopolymer ranges from about 40,000 to about 500,000 grams per more, or from about 50,000 to about 250,000 grams per mole, or from about 50,000 to about 100,000 grams per mole. As used herein, the term "molecular weight" means weight average molecular weight. Methods for determining average molecular weight of biopolymers include low angle laser light scattering (LLS) and Size Exclusion Chromatography (SEC). In performing low angle LLS, a dilute solution of the polysaccharide, typically 2% or less, is placed in the path of a monochromatic laser. Light scattered from the sample hits the detector, which is positioned at a low angle relative to the laser source. Fluctuation in scattered light over time is correlated with the average molecular weight of the polysaccharide in solution. In performing SEC measurements, again a dilute solution of biopolymer, typically 2% or less, is injected into a packed column. The polysaccharide is separated based on the size of the dissolved polymer molecules and compared with a series of standards to derive the molecular weight.

A hydrophobically modified biopolymer material for incorporation into a container having at least two compartments can be an aqueous formulation. In some embodiments, the hydrophobically-modified chitosan is present at about 0.1% to about 2% by weight, or from about 0.1 to about 1% of the total weight of the solution. In some embodiments, the hydrophobically-modified chitosan is present at about 0.2% to about 0.8% by weight relative to the total weight of the solution. In some embodiments, the hydrophobically-modified chitosan is present at 0.3% by weight to about 0.7% by weight (e.g., about 0.5% or about 0.6% by weight).

Hydrophobic moieties can be independently selected from saturated hydrocarbons (e.g., alkanes), which are optionally acyl groups, and unsaturated hydrocarbons (e.g., alkenes, alkynes), which may be linear, branched or cyclic. In some embodiments, the hydrophobic moieties include aromatic and/or polyaromatic hydrocarbons. In some embodiments, the hydrophobic moiety is a hydrocarbon having from about 4 to about 100 carbon atoms, or from about 8 to about 60 carbon atoms, or from about 8 to about 28 carbon atoms, or from about 8 to about 36 carbons, or from about 8 to about 18 carbon atoms, or from about 8 to about 20 carbon atoms. The hydrophobic groups may be linear hydrocarbons having about 12 to about 18 carbon atoms on average, such as an average of from 14 to 16 carbon atoms on average.

In some embodiments, the hydrophobically modified chitosan contains linear, saturated C8, C10, or C12 hydrocarbon moieties, which provide good foaming properties. In some embodiments, the integrity of the foam is improved with addition of from 5% to about 40% (relative to the total number of hydrophobic grafts) of C16 or C18 linear, hydrocarbon

moieties. In some embodiments, the hydrophobically-modified chitosan has from 10% to about 30% C16 or C18 hydrocarbon moieties. In some embodiments, the hydrophobic moieties are C12 and C18, where the majority are C12.

The hydrophobic substituents may be a hydrocarbon group having from about 8 to about 18 carbon atoms attached to the backbone of the one biopolymer, and in some embodiments comprises an alkyl group. In some embodiments, the hydrocarbon group comprises an arylalkyl group. As used herein, the term "arylalkyl group" means a group containing both aromatic and aliphatic structures.

The hemostat product may comprise numerous hydrophobically modified biopolymer compounds. These compounds comprise a biopolymer (such as chitosan) backbone that includes a hydrophilically reactive functional group (e.g., amino groups) that binds with the hydrophilically reactive head groups (e.g., carbonyl functional group) of an amphiphilic compound (e.g., aldehyde), to form the hm-chitosan or other hm-polymer. The head group is further associated with a hydrophobic tail group. In the current embodiment, the hydrophobic tail may be, for example, a hydrocarbon. Thus, a hydrophobic tail is associated with the biopolymer backbone providing the hydrophobic modification to the molecule that extends from the backbone and may interact with a surrounding environment in numerous ways, such as through hydrophobic interaction with materials.

Examples of procedures for modifying polymers are as follows.

Alginates can be hydrophobically modified by exchanging their positively charged counterions (e.g. Na+) with tertiary-butyl ammonium (TBA) ions using a sulfonated ion exchange resin. The resulting TBA-alginate is dissolved in dimethylsulfoxide (DMSO) where reaction occurs between alkyl (or aryl) bromides and the carboxylate groups along the alginate backbone. Alginate can also be modified by fatty amine groups (e.g. dodecyl amine), followed by addition of 1-ethyl-3-(3-dimethylaminopropyl) carbodiimide, via EDC coupling.

Cellulosics can be hydrophobically modified by first treating the cellulosic material with a large excess highly basic aqueous solution (e.g. 20 wt % sodium hydroxide in water). The alkali cellulose is then removed from solution and vigorously mixed with an emulsifying solution (for example, oleic acid) containing the reactant, which is an alkyl (or aryl) halide (e.g. dodecyl bromide).

Chitosans can be hydrophobically modified by reaction of alkyl (or aryl) aldehydes with primary amine groups along the chitosan backbone in a 50/50 (v/v)% of aqueous 0.2 M acetic acid and ethanol. After reaction, the resulting Schiff bases, or imine groups, are reduced to stable secondary amines by dropwise addition of the reducing agent sodium cyanoborohydride.

The degree of substitution of the hydrophobic substituent on the polymer is up to 50% of available functional groups, for example, amines in the case of chitosan. For example, the hydrophobic substituent can be added to from 10 to 50% of available amines, or from 20 to 50% of available amine, or from 30 to 50% of available amines. It is contemplated that more than one particular hydrophobic substituent may be substituted onto the polymer, provided that the total substitution level is substantially within the ranges set forth above.

In some embodiments, the hydrophobic substituent is derived from an amphiphilic compound, meaning it is composed of a hydrophilic Head group and a hydrophobic Tail group. The Head group binds with the polymer and positions the Tail group to extend from the backbone of the polymer scaffold. This makes the hydrophobic Tail group available for hydrophobic interactions. The Tail group is a hydrocarbon of various forms.

Hydrocarbons that find use in accordance with this disclosure may be classified as saturated hydrocarbons, unsaturated hydrocarbons, and aromatic hydrocarbons. From this basic classification system there exist many derivatives and further types of compounds that build therefrom. For example, numerous and varied compounds include more than one aromatic ring and are generally referred to as polyaromatic hydrocarbons (PAH). In some embodiments, the hydrophobic moiety is aliphatic. Aliphatic compounds, carbon atoms can be joined together in straight chains, branched chains, or rings (in which case they are called alicyclic). They can be joined by single bonds (alkanes), double bonds (alkenes), or triple bonds (alkynes). Besides hydrogen, other elements can be bound to the carbon chain, the most common being oxygen, nitrogen, sulfur, and chlorine. Those of ordinary skill in the art will recognize that other molecules may also be bound to the carbon chains and that compounds of such heteroatomic structure are contemplated as falling within the scope of the current invention.

The hydrophobic Tail group of the amphiphilic compound bound to the polymer backbone of the current invention is capable of branching and/or allowing the inclusion of side chains onto its carbon backbone. It may be understood that the strength of the hydrophobic interaction is based upon the available amount of "hydrophobes" that may interact amongst themselves or one another. Thus, it may further promote the hydrophobic effect by increasing the amount of and/or hydrophobic nature of the hydrophobic Tail group that is interacting. For instance, a hydrophobic Tail group, which in its original form may include a hydrocarbon chain, may promote an increase in its hydrophobicity (ability to hydrophobically bond and strength of hydrophobic interaction) by having a hydrophobic side chain attach to one of the carbons of its carbon backbone.

In some embodiments, the current invention contemplates the use of various molecules and/or compounds that may increase hemorrhage control, durability, water repellent properties, and/or flexibility of the hemostat product. The side chains may be linear chains, aromatic, aliphatic, cyclic, polycyclic, or any various other types of hydrophobic

side chains as contemplated by those skilled in the art.

In some embodiments, the hydrophobic grafts include an alicyclic, cycloalkane, or cycloalkene. For example, the hydrophobic group may be both aliphatic and cyclic with or without side chains attached. In some embodiments, the cyclic groups are carbocyclic groups, which may be saturated or unsaturated (aromatic or non-aromatic).

In some embodiments, the hydrophobic grafts include aromatic hydrocarbon, or polycyclic aromatic hydrocarbon, or heterocyclic moieties. Heterocyclic groups may include, in addition to carbon, at least one atom such as nitrogen, oxygen, or sulfur, as part of the ring. Examples include pyridine ($C_5H_5N$), Pyrimidine ($C_4H_4N_2$) and Dioxane.

Some of the contemplated hydrophobic side chains may include the following:

Table 1: Linear Alkanes

| Number of C Atoms | Formula | Common Name |
|---|---|---|
| 1 | $CH_4$ | Methane |
| 2 | C2H6 | Ethane |
| 3 | $C_3H_8$ | Propane |
| 4 | $C_4H_{10}$ | n-Butane |
| 5 | $C_5H_{12}$ | n-Pentane |
| 6 | $C_6H_{14}$ | n-Hexane |
| 7 | $C_7H_{16}$ | n-Heptane |
| 8 | $C_8H_{18}$ | n-Octane |
| 9 | $C_9H_{20}$ | n-Nonane |
| 10 | $C_{10}H_{22}$ | n-Decane |
| 11 | $C_{11}H_{24}$ | n-Undecane |
| 12 | $C_{12}H_{26}$ | n-Dodecane |
| 13 | $C_{13}H_{28}$ | n-Trideacane |
| 14 | $C_{14}H_{30}$ | n-Tetradecane |
| 15 | $C_{15}H_{32}$ | n-Pentadecane |
| 16 | $C_{16}H_{34}$ | n-Hexadecane |
| 17 | $C_{17}H_{36}$ | n-Heptadecane |
| 18 | $C_{18}H_{38}$ | n-Octadecane |
| 19 | $C_{19}H_{40}$ | n-Nonadecane |
| 20 | $C_{20}H_{42}$ | n-Eicosane |
| 21 | $C_{21}H_{44}$ | n-Heneicosane |
| 22 | $C_{22}H_{46}$ | n-Docosane |
| 23 | $C_{23}H_{48}$ | n-Tricosane |
| 24 | $C_{24}H_{50}$ | n-Tetracosane |
| 25 | $C_{25}H_{52}$ | n-Pentacosane |
| 26 | $C_{26}H_{54}$ | n-Hexacosane |
| 27 | $C_{27}H_{56}$ | n-Heptacosane |
| 28 | $C_{28}H_{58}$ | n-Octacosane |
| 29 | $C_{29}H_{60}$ | n-Nonacosane |
| 30 | $C_{30}H_{62}$ | n-Triacontane |
| 31 | $C_{31}H_{64}$ | n-Hentraiacontane |
| 32 | $C_{32}H_{66}$ | n-Dotriacontane |

(continued)

| Number of C Atoms | Formula | Common Name |
|---|---|---|
| 33 | $C_{33}H_{68}$ | n- Tritriacontane |
| 34 | $C_{34}H_{70}$ | n-Tetratriacontane |
| 35 | $C_{35}H_{72}$ | n-Pentatriacontane |
| 36 | $C_{36}H_{74}$ | n-Hexatriacontane |
| 37 | $C_{37}H_{76}$ | n-Heptatriacontane |
| 38 | $C_{38}H_{78}$ | n-Octatriacontane |
| 39 | $C_{39}H_{80}$ | n-Nonactriacontane |
| 40 | $C_{40}H_{82}$ | n-Tetracontane |
| 41 | $C_{41}H_{84}$ | n-Hentatetracontane |
| 42 | $C_{42}H_{86}$ | n-Dotetracontane |
| 43 | $C_{43}H_{88}$ | n- Tritetracontane |
| 44 | $C_{44}H_{90}$ | n- Tetratetracontane |
| 45 | $C_{45}H_{92}$ | n-Pentatetracontane |
| 46 | $C_{46}H_{94}$ | n-Hexatetracontane |
| 47 | $C_{47}H_{96}$ | n-Heptatetracontane |
| 48 | $C_{48}H_{98}$ | n -Octatetracontane |
| 49 | $C_{49}H_{100}$ | n-Nonatetracontane |
| 50 | $C_{50}H_{102}$ | n-Pentacontane |
| 51 | $C_{51}H_{104}$ | n-Henpentacontane |
| 52 | $C_{52}H_{106}$ | n-Dopentacontane |
| 53 | $C_{53}H_{108}$ | n-Tripentacontane |
| 54 | $C_{54}H_{110}$ | n-Tetrapentacontane |
| 55 | $C_{55}H_{112}$ | n-Pentapentacontane |
| 56 | $C_{56}H_{114}$ | n-Hexapentacontane |
| 57 | $C_{57}H_{116}$ | n-Heptapentacontane |
| 58 | $C_{58}H_{118}$ | n-Octapentacontane |
| 59 | $C_{59}H_{120}$ | n-Nonapentacontane |
| 60 | $C_{60}H_{122}$ | n-Hexacontane |
| 61 | $C_{61}H_{124}$ | n-Henhexacontane |
| 62 | $C_{62}H_{126}$ | n-Dohexacontane |
| 63 | $C_{63}H_{128}$ | n-Trihexacontane |
| 64 | $C_{64}H_{130}$ | n-Tetrahexacontane |
| 65 | $C_{65}H_{132}$ | n-Pentahexacontane |
| 66 | $C_{66}H_{134}$ | n-Hexahexacontane |
| 67 | $C_{67}H_{136}$ | n-Heptahexacontane |
| 68 | $C_{68}H_{138}$ | n-Octahexacontane |
| 69 | $C_{69}H_{140}$ | n-Nonahexacontane |
| 70 | $C_{70}H_{142}$ | n-Heptacontane |

(continued)

| Number of C Atoms | Formula | Common Name |
|---|---|---|
| 71 | $C_{71}H_{144}$ | n-Henheptacontane |
| 72 | $C_{72}H_{146}$ | n-Doheptacontane |
| 73 | $C_{73}H_{148}$ | n-Triheptacontane |
| 74 | $C_{74}H_{150}$ | n-Tetraheptacontane |
| 75 | $C_{75}H_{152}$ | n-Pentaheptacontane |
| 76 | $C_{76}H_{154}$ | n-Hexaheptacontane |
| 77 | $C_{77}H_{156}$ | n-Heptaheptacontane |
| 78 | $C_{78}H_{158}$ | n-Octaheptacontane |
| 79 | $C_{79}H_{160}$ | n-Nonaheptacontane |
| 80 | $C_{80}H_{162}$ | n-Otcacontane |
| 81 | $C_{81}H_{164}$ | n-Henoctacontane |
| 82 | $C_{82}H_{166}$ | n-Dooctacontane |
| 83 | $C_{83}H_{168}$ | n-Trioctacontane |
| 84 | $C_{84}H_{170}$ | n-Tetraoctacontane |
| 85 | $C_{85}H_{172}$ | n-Pentaoctacontane |
| 86 | $C_{86}H_{174}$ | n-Hexaoctacontane |
| 87 | $C_{87}H_{176}$ | n-Heptaoctacontane |
| 88 | $C_{88}H_{178}$ | n-Octaoctacontane |
| 89 | $C_{89}H_{180}$ | n-Nonaoctacontane |
| 90 | $C_{90}H_{182}$ | n-Nonacontane |
| 91 | $C_{91}H_{184}$ | n-Hennonacontane |
| 92 | $C_{92}H_{186}$ | n-Dononacontane |
| 93 | $C_{93}H_{188}$ | n-Trinonacontane |
| 94 | $C_{94}H_{190}$ | n-Tetranonacontane |
| 95 | $C_{95}H_{192}$ | n-Pentanonacontane |
| 96 | $C_{96}H_{194}$ | n-Hexanonacontane |
| 97 | $C_{97}H_{196}$ | n-Heptanonacontane |
| 98 | $C_{98}H_{198}$ | n-Octanonacontane |
| 99 | $C_{99}H_{200}$ | n-Nonanonacontane |
| 100 | $C_{100}H_{202}$ | n-Hectane |
| 101 | $C_{101}H_{204}$ | n-Henihectane |
| 102 | $C_{102}H_{206}$ | n-Dohectane |
| 103 | $C_{103}H_{208}$ | n-Trihectane |
| 104 | $C_{104}H_{210}$ | n-Tetrahectane |
| 105 | $C_{105}H_{212}$ | n-Pentahectane |
| 106 | $C_{106}H_{214}$ | n-Hexahectane |
| 107 | $C_{107}H_{216}$ | n-Heptahectane 10 |
| 108 | $C_{108}H_{218}$ | n-Octahectane |

(continued)

| Number of C Atoms | Formula | Common Name |
|---|---|---|
| 109 | $C_{109}H_{220}$ | n-Nonahectane |
| 110 | $C_{110}H_{222}$ | n-Decahectane |
| 111 | $C_{111}H_{224}$ | n-Undecahectane |

Table 2: Cyclic Compounds

| Polycyclic Compounds | Sub-Types | Example Compounds |
|---|---|---|
| Bridged Compound - compounds which contain interlocking rings | Bicyclo compound | adamantine |
| | | amantadine |
| | | biperiden |
| | | memantine |
| | | methenamine |
| | | rimantadine |
| Macrocyclic Compounds | Calixarene | |
| | Crown Compounds | |
| | Cyclodextrins | |
| | Cycloparaffins | |
| | Ethers, Cyclic | |
| | Lactans, macrocyclic | |
| | Macrolides | |
| | Peptides, Cyclic | |
| | Tetrapyrroles | |
| | Trichothecenes | |
| Polycyclic Hydrocarbons, Aromatic | Acenaphthenes | |
| | Anthracenes | |
| | Azulenes | |
| | Benz(a)anthracenes | |
| | Benzocycloheptenes | |
| | Fluorenes | |
| | Indenes | |
| | Naphthalenes | |
| | Phenalenes | |
| | Phenanthrenes | |
| | Pyrenes | |
| | Spiro Compounds | |

(continued)

| Polycyclic Compounds | Sub-Types | Example Compounds |
|---|---|---|
| Steroids | Androstanes | |
| | Bile Acids and Salts | |
| | Bufanolides | |
| | Cardanolides | |
| | Cholanes | |
| | Choestanes | |
| | Cyclosteroids | |
| | Estranes | |
| | Gonanes | |
| | Homosteroids | |
| | Hydroxysteroids | |
| | Ketosteroids | |
| | Norsteroids | |
| | Prenanes | |
| | Secsteroids | |
| | Spirostans | |
| | Steroids, Brominated | |
| | Steroids, Chlorinated | |
| | Steroids, Fluorinated | |
| | Steroids, Heterocyclic | |

Any aspect or embodiment described herein can be combined with any other aspect or embodiment as disclosed herein.

Other aspects and embodiments of the invention will be apparent to the skilled artisan from this disclosure.

The invention will be further described in the following examples, which do not limit the scope of the invention described in the claims.

EXAMPLES

Example 1: Designing hm-chitosan foams

To establish a robust foam to treat blood loss (e.g., epistaxis), a dual-barrel syringe with a static mixing tip was used to stabilize carbon dioxide ($CO_2$) gas with hydrophobically-modified chitosan. $CO_2$ is generated by the reaction between acetic acid and sodium bicarbonate:

$$CH_3COOH + NaHCO_3 \rightarrow CH_3COONa + CO_{2(g)} + H_2O$$

Since this foam system is intended for use as a hemostat (e.g., for epistaxis), it is important to minimize the acid content and maintain an appropriate salt concentration.

An image of the syringe system is shown in Figure 1A and Figure IB. In side A, HmC is dissolved in AcOH, in this case 0.38M AcOH; side B contains an equal volume of 0.3M NaBicarb. In the examples of this disclosure, the formulations are specific to a 1:1 syringe barrel ratio with 2x8mm tip (#4B19; manufacturer: PlasPak; as distributed by DeDoes Inc). Other tip sizes and syringe volume/mix ratios can be employed. For example, 2mL 0.38M AcOH mixed with 2mL 0.3M NaBicarb theoretically produces 7.46mL $CO_2$ gas, and the aqueous phase contains 0.15M sodium acetate (NaAcetate) and 0.04M AcOH leftover. Accordingly, the resultant salt concentration was enough to osmotically balance

the natural salt content of blood plasma (0.15M NaCl), and the final acid content was under 0.05M, which was determined to be appropriate for blood, since when mixed with blood, the sample remained bright red over long periods of time rather than turning dark, which would have indicated a lower level of oxygen in the blood. An approximation of the final pH of the aqueous phase of the foam can be calculated from pKa values for AcOH and NaAcetate; in the experiments shown, the pH was 5.3.

Hm-Chitosan generally requires an acidic environment to dissolve. HmAlginate (HmA) does not have this issue, and thus potentially could be included in side B. For hm-chitosan, the pH of side A should be larger than pH 3 to avoid precipitate. Further, the viscosity of the solutions must be sufficiently low to allow operative syringing and foam formation.

Example 2: Characterization of static mixer foams

This example analyzes the mechanical properties of the hm-chotosan foam.

Generally, the bulk modulus of the foam is primarily affected by the enclosed gas phase while the shear modulus is dictated by surface tension and bubble size. The bulk modulus can be probed via dilatation; as each dilation provides information regarding the adsorption/desorption of surfactants. Shear rheology probes the interaction between surfactants. Additionally, bubble size, or the surface tension/bubble size ratio, also contributes to the shear modulus; however, surface tension, surface elasticity (surface elasticity can be measured using a rheometer with special geometry), and viscosity can also each affect bubble size. In general, a smaller bubble size yields a longer drainage time, and therefore, longer stability. The capillary number is a property that relates contributions from the viscous forces and surface tension. The hmpolymers disclosed herein seem likely to most resemble proteins, in that they have both hydrophobic and hydrophilic components and are large molecules that have self-assembly properties. Surface tension of proteins increases with increasing molecular weight, however small, disordered, flexible proteins are known to be good surfactants. Moreover, the effect of salts in hmpolymer solutions affect viscosity and surface tension by screening electrostatic repulsions allowing for a more compact molecular structure. These are some of the key mechanical properties of foam.

Other key parameters for foam formation (i.e., foamability) include foam capacity (*FC*), maximum foam density (*MD*), percent volumetric foam stability (%*FVS*) and percent liquid stability (*%FLS*), each are presented below. For clarification, $V_{foam0}$ is the volume of formed foam, $V_{gas}$ is the volume of gas introduced for the formation of the foam, $V_{liq0}$ is the volume of liquid entrapped in the formed foam, and $V_{foam}$ and $V_{liq}$ are the volumes of foam and entrapped liquid at time t, respectively. These are qualitative parameters to describe foams, as represented by the following formulas:

$$FC = {V_{foam0}}/{V_{gas}}$$

$$MD = {V_{liq0}}/{V_{foam0}}$$

$$\%FVS = \left({V_{foam}}/{V_{foam0}}\right) \times 100$$

$$\%FLS = \left({V_{liq}}/{V_{liq0}}\right) \times 100$$

Based on the above overview of foam properties, one would hypothesize that the decrease in surface tension caused by a surfactant has a significant impact on foamability. However, based on the preliminary surface tension measurements completed for Hmalginate and Hm-chitosan, the decrease in surface tension appeared to be inadequate to stabilize gas. As shown in Figure 2, the lowest surface tension measured is about 69 mNm, where pure water is 72.5 and pure acetone is 23.5. To determine whether this was a discrepancy in the surface tension measurement technique, or whether it was the actual surface tension measurement, dynamic measurements were conducted with a hanging drop tensiometer (i.e., Fisher Scientific Surface Tensiometer 21 with a bent platinum ring, which was able to measure pure water and acetone within 5% accuracy).

Typical mechanical behavior of foams follows that of Bingham plastics, where elastic deformation at low strain leads into plastic deformation (from bubble rearrangement) and flow. Under flow, it is possible for bubbles to rearrange and segregate by size. Foam stability is controlled by drainage, bubble coalescence, and bubble disproportionation. For

the static mixer foams stabilized with Hm-chitosan, typical dynamic rheological behavior is shown in Figure 3. Figure 3 shows that the foam exhibits gel-like behavior compared to the pure solution of an equivalent composition. Figure 6 shows shear thinning behavior at a strain of about 10%.

Monitoring foam height, or shear modulus, over time allows for an indication of foam stability. Figure 5 shows a time sweep for an HmC foam where the storage modulus remains fairly constant over 15 minutes, whereas the loss modulus decreases by about half after about 10 minutes. A decrease in G" with steady G' may be indicative of bubble coalescence and drainage, as the overall liquid content decreases and bubble size increases.

Presented in this example are results for Hm-chitosan hybrid (5% C12 1% C18) using a double-barrel syringe with a static mixing tip to generate a foam from the reaction between AcOH and NaBicarb.

Regarding the mixture of foams with blood, the physical manipulation of a foam changes the number of bubbles as well as size. With this in view, the static mixer foams of the present disclosure are able to form in situ, allowing the formation of the foam within a blood sample. This may be a way to create foam/blood mixtures without compromising the natural foam structure. Figure 4 shows that the G' is preserved when mixing the foam with blood and not when mixed with PBS. This is important. In this case, the effect from salts are removed based on the fact that salt is part of the foam formulation. One hypothesis is that the interaction at the liquid interface is controlling the modulus. Indeed, it has been reported that shear modulus is controlled by surface tension, as well as the interactions between surface agents can be probed using shear. This may be a demonstration of the interaction between blood cells and Hm-chitosan, such as gelation or aggregation. Macroscopic cell clusters may be forming when mixing Hm-chitosan with blood, so there may be some interaction between the two components.

Example 3: The Efficacy of HM-chitosan in an *in vivo* Rat Anterior Epistaxis Model

About 90% of epistaxis cases are anterior epistaxis caused by hemorrhage from vessels in Kiesselbach's plexus in Little's area. Current treatments for epistaxis include embolization, arterial ligation, nasal packing and hemostatic agents such as chitosan, fibrin and floseal. However, there are many drawbacks associated with current treatments, including facial pain, soft tissue necrosis, infections, nasal septum perforation, and cost effectiveness.

To evaluate the efficacy of HM-chitosan *in vivo,* a rat anterior epistaxis model was used that enabled the measurement of blood loss and the time needed to reach hemostasis (Figure 8). In this model, control rats included rats that did not receive any treatment, rats that only received a saline solution (Figure 9), and rats that received a gauze pack only. The treatment rats included rats that were treated with HM-chitosan foam, rats that were treated with HM-chitosan gauze packing (Figure 10), or rats that were treated with only chitosan packing (Figure 8). In this experiment, the rats were initially anesthetized with a combination of ketamine and xylaxine, which was then followed up with intravenous (IV) administration of heparin (640IU/kg). The rats then received a 2mm surgical punch, and measurements were then taken to analyze blood loss and the time needed to reach hemostasis. A total of 28 were tested in this model, although 12 were excluded based on the criteria.

Results demonstrated that rats treated with either the hm-chitosan gauze or the hm-chitosan foam experienced a significant decrease in blood loss compared to either the no treatment control rats or control gauze rats (Figures 10 and 11). In addition, rats treated with either the hm-chitosan gauze or the hm-chitosan foam had reduced blood loss per minute post nasal punch when compared to either the no treatment control rats or control gauze rats (Figure 12). The time needed to reach the first blood droplet was shorter for the rats treated with either the hm-chitosan gauze or the hm-chitosan foam compared to either the no treatment control rats or control gauze rats (Figure 13). Furthermore, the time needed for total hemostasis was significantly lower for the rats treated with either the hm-chitosan gauze or the hm-chitosan foam compared to either the no treatment control rats or control gauze rats.

## Claims

1. A hemostat product comprising a container having at least two compartments each containing a releasable, flowable product: wherein a first compartment comprises a solution of an acidified hydrophobically-modified chitosan that gels in the presence of blood, and a second compartment comprises a solution of a bicarbonate or carbonate salt at an alkaline pH and optionally a negatively-charged polymer,
   wherein the hydrophobically-modified chitosan includes hydrophobic grafts larger than C12, and from 5% to about 40% of the hydrophobic grafts are C16 or C18.

2. The product of claim 1, wherein the modified chitosan has a level of deacetylation of from about 40 to about 90%.

3. The product of claim 2, wherein the modified chitosan has from about 10% to about 50% of functional groups occupied by a hydrophobic group.

**4.** The product of claim 3, wherein the hydrophobic groups comprise saturated hydrocarbons, which are optionally acyl groups.

**5.** The product of claim 4, wherein the hydrophobic groups are independently selected from linear hydrocarbons having from 8 to about 18 carbon atoms.

**6.** The product of any one of claims 1 to 5, wherein the modified chitosan is an aqueous formulation, optionally wherein the modified chitosan is present at 0.1 to about 2% by weight of the solution in the first compartment.

**7.** The product of any one of claims 1 to 6, wherein the modified chitosan is formulated at a pH of from 2.5 to 4.5.

**8.** The product of claim 7, wherein the modified chitosan is formulated with lactic acid, ascorbic acid, or citric acid.

**9.** The product of any one of claims 1 to 8, wherein the second compartment comprises a solution comprising sodium bicarbonate, optionally wherein the bicarbonate solution is from about 0.2M to about 0.5M sodium bicarbonate.

**10.** The product of any one of claims 1 to 8, wherein the bicarbonate or carbonate salt is formulated at pH 8 to 10, optionally further comprising a negatively-charged polymer in the second compartment, optionally wherein the negatively-charged polymer is alginate, pectin, dextran, carboxymethylcellulose, or xanthan gum, and optionally wherein the negatively charged polymer is hydrophobically-modified alginate.

**11.** The product of claim 10, wherein the container is a double barrel syringe, wherein releasing the contents results in mixing of the releasable, flowable products, and wherein the combination of the flowable products results in production of $CO_2$, and foaming of the hydrophobically-modified chitosan composition.

**12.** A product according to any one of claims 1-11 for use in the treatment of a bleeding wound, wherein the contents of the product are released on said bleeding wound.

**13.** The product for use of claim 12, wherein the wound is an internal wound or a wound in a body cavity.

**14.** The product for use of claim 13, wherein the bleeding wound is epistaxis and the contents of the product are released into one or more nostrils.

**15.** The product for use of any one of claims 12 to 14, wherein the hydrophobically-modified chitosan provides hemostasis within about 2 minutes, or within about 1 minute.

## Patentansprüche

**1.** Ein Hämostase-Produkt, umfassend einen Behälter mit mindestens zwei Kompartimenten, wobei jedes ein freisetzbares, fließfähiges Produkt enthält: wobei ein erstes Kompartiment eine Lösung eines azidifizierten hydrophob-modifizierten Chitosan umfasst, das in der Gegenwart von Blut geliert, und ein zweites Kompartiment eine Lösung eines Bicarbonat- oder Carbonatsalzes bei alkalischen pH und optional ein negativ-geladenes Polymer umfasst, wobei das hydrophob-modifizierte Chitosan hydrophobe Grafts größer als C12 einschließt und etwa 5% bis etwa 40% hydrophobe Grafts C16 oder C18 sind.

**2.** Das Produkt nach Anspruch 1, wobei das modifizierte Chitosan Deacetylierungslevel von etwa 40 bis etwa 90% hat.

**3.** Das Produkt nach Anspruch 2, wobei das modifizierte Chitosan etwa 10% bis etwa 50% der funktionellen Gruppen durch hydrophobe Gruppen besetzt hat.

**4.** Das Produkt nach Anspruch 3, wobei die hydrophoben Gruppen gesättigte Kohlenwasserstoffe umfassen, die optional Acylgruppen sind.

**5.** Das Produkt nach Anspruch 4, wobei die hydrophoben Gruppen unabhängig ausgewählt sind aus linearen Kohlenwasserstoffen mit 8 bis etwa 18 Kohlenstoffatomen.

**6.** Das Produkt nach einem der Ansprüche 1 bis 5, wobei das modifizierte Chitosan eine wässrige Formulierung ist,

optional wobei das modifizierte Chitosan zu 0,1 bis etwa 2 Gew.% der Lösung im ersten Kompartiment vorliegt.

**7.** Das Produkt nach einem der Ansprüche 1 bis 6, wobei das modifizierte Chitosan bei einem pH von 2,5 bis 4,5 formuliert ist.

**8.** Das Produkt nach Anspruch 7, wobei das modifizierte Chitosan mit Milchsäure, Ascorbinsäure oder Zitronensäure formuliert ist.

**9.** Das Produkt nach einem der Ansprüche 1 bis 8, wobei das zweite Kompartiment eine Lösung umfassend Natriumbicarbonat umfasst, optional wobei die Bicarbonatlösung 0,2M bis etwa 0,5M Natriumbicarbonat ist.

**10.** Das Produkt nach einem der Ansprüche 1 bis 8, wobei das Bicarbonat- oder Carbonatsalz bei pH 8 oder 10 formuliert ist, optional ferner umfassend ein negativ-geladenes Polymer im zweiten Kompartiment, optional wobei das negativ-geladene Polymer Alginat, Pektin, Dextran, Carboxymethylcellulose oder Xanthangummi ist und optional wobei das negativ-geladene Polymer hydrophob-modifiziertes Alginat ist.

**11.** Das Produkt nach Anspruch 10, wobei der Behälter eine Doppellaufspritze ist, wobei das Freisetzen der Inhaltsstoffe zum Mischen der freisetzbaren, fließfähigen Produkte führt, und wobei die Kombination von fließfähigen Produkten zur Produktion von $CO_2$ und dem Schäumen der hydrophob-modifizierten Chitosan-Zusammensetzung führt.

**12.** Ein Produkt nach einem der Ansprüche 1-11 zur Verwendung bei der Behandlung einer blutenden Wunde, wobei die Inhaltsstoffe des Produkts auf jene blutende Wunde freigesetzt werden.

**13.** Das Produkt zur Verwendung nach Anspruch 12, wobei die Wunde eine innerliche Wunde oder eine Wunde in der Körperhöhle ist.

**14.** Das Produkt zur Verwendung nach Anspruch 13, wobei die blutende Wunde Nasenbluten ist und die Inhaltsstoffe des Produkts in ein oder mehrere Nasenlöcher freigesetzt werden.

**15.** Das Produkt zur Verwendung nach einem der Ansprüche 12 bis 14, wobei das hydrophob-modifizierte Chitosan Hämostase innerhalb von etwa 2 Minuten oder innerhalb von etwa 1 Minute bereitstellt.

**Revendications**

**1.** Produit hémostatique comprenant un récipient ayant au moins deux compartiments contenant chacun un produit fluide, libérable : dans lequel un premier compartiment comprend une solution d'un chitosane acidifié hydrophobiquement modifié qui se gélifie en présence de sang, et un second compartiment comprend une solution d'un sel de bicarbonate ou de carbonate à un pH alcalin et facultativement un polymère à charge négative,
dans lequel le chitosane hydrophobiquement modifié inclut des greffes hydrophobes supérieures à C12, et d'environ 5 % à environ 40 % des greffes hydrophobes sont en C16 ou C18.

**2.** Produit selon la revendication 1, dans lequel le chitosane modifié a un niveau de désacétylation d'environ 40 à environ 90 %.

**3.** Produit selon la revendication 2, dans lequel le chitosane modifié a d'environ 10 % à environ 50 % de groupes fonctionnels occupés par un groupe hydrophobe.

**4.** Produit selon la revendication 3, dans lequel les groupes hydrophobes comprennent des hydrocarbures saturés, qui sont facultativement des groupes acyle.

**5.** Produit selon la revendication 4, dans lequel les groupes hydrophobes sont indépendamment sélectionnés parmi des hydrocarbures linéaires ayant de 8 à environ 18 atomes de carbone.

**6.** Produit selon l'une quelconque des revendications 1 à 5, dans lequel le chitosane modifié est une formulation aqueuse, facultativement dans lequel le chitosane modifié est présent à 0,1 à environ 2 % en poids de la solution dans le premier compartiment.

7. Produit selon l'une quelconque des revendications 1 à 6, dans lequel le chitosane modifié est formulé à un pH de 2,5 à 4,5.

8. Produit selon la revendication 7, dans lequel le chitosane modifié est formulé avec de l'acide lactique, de l'acide ascorbique, ou de l'acide citrique.

9. Produit selon l'une quelconque des revendications 1 à 8, dans lequel le second compartiment comprend une solution comprenant du bicarbonate de sodium, facultativement dans lequel la solution de bicarbonate est d'environ 0,2 M à environ 0,5 M de bicarbonate de sodium.

10. Produit selon l'une quelconque des revendications 1 à 8, dans lequel le sel de bicarbonate ou de carbonate est formulé à pH 8 à 10, comprenant facultativement en outre un polymère à charge négative dans le second compartiment, facultativement dans lequel le polymère à charge négative est un alginate, une pectine, un dextrane, une carboxyméthylcellulose, ou de la gomme de xanthane, et facultativement dans lequel le polymère à charge négative est un alginate hydrophobiquement modifié.

11. Produit selon la revendication 10, dans lequel le récipient est une seringue à deux cylindres, dans lequel la libération des contenus résulte en le mélange des produits fluides, libérables, et dans lequel la combinaison des produits fluides résulte en une production de $CO_2$, et un moussage de la composition de chitosane hydrophobiquement modifié.

12. Produit selon l'une quelconque des revendications 1 à 11 pour son utilisation dans le traitement d'une plaie hémorragique, dans lequel les contenus du produit sont libérés sur ladite plaie hémorragique.

13. Produit pour son utilisation selon la revendication 12, dans lequel la plaie est une plaie interne ou une plaie dans une cavité corporelle.

14. Produit pour son utilisation selon la revendication 13, dans lequel la plaie hémorragique est une épistaxis et les contenus du produit sont libérés dans une narine ou plus.

15. Produit pour son utilisation selon l'une quelconque des revendications 12 à 14, dans lequel le chitosane hydrophobiquement modifié permet une hémostase en environ 2 minutes, ou en environ 1 minute.

FIGURE 1A

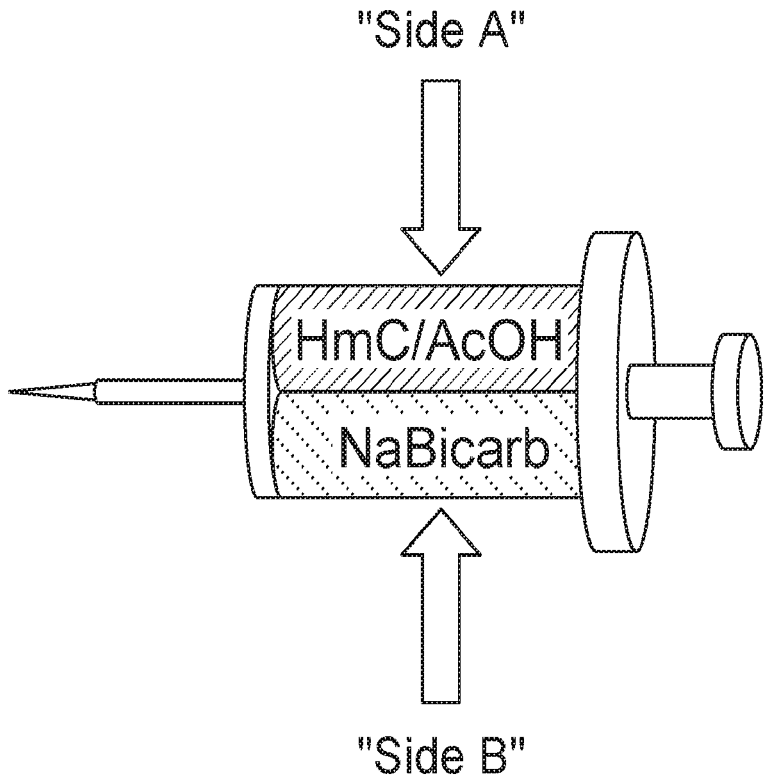

FIGURE 1B

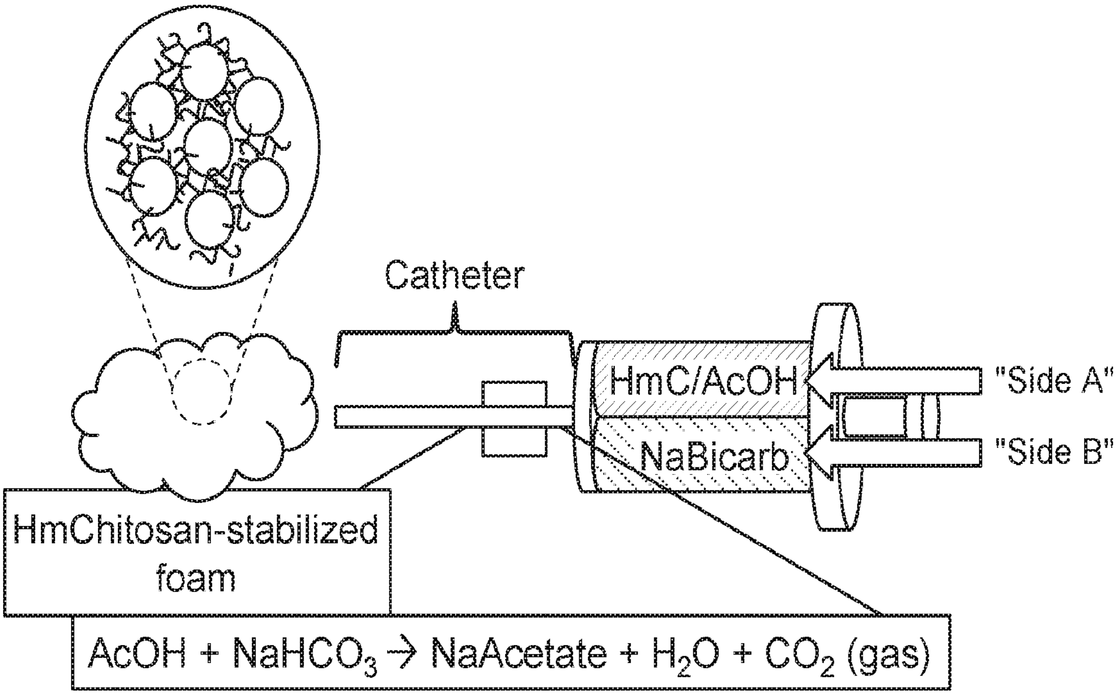
Catheter
HmC/AcOH
NaBicarb
"Side A"
"Side B"
HmChitosan-stabilized foam
AcOH + NaHCO3 → NaAcetate + H2O + CO2 (gas)

FIGURE 2

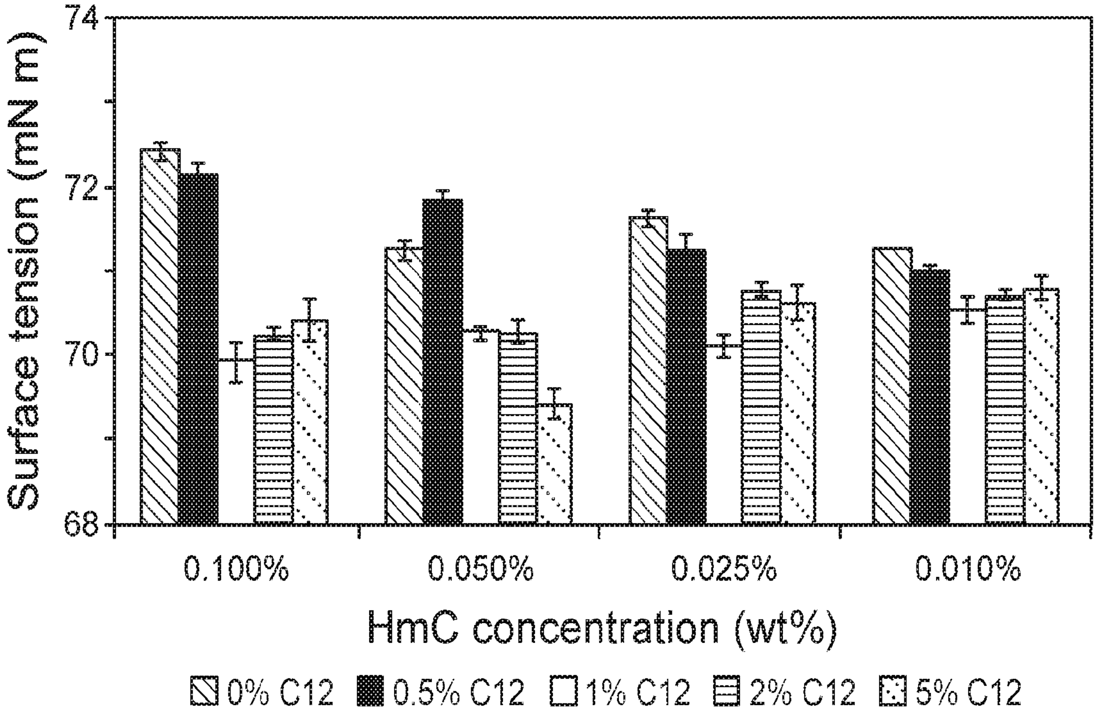
74
72
70
68
Surface tension (mN m)
0.100%
0.050%
0.025%
0.010%
HmC concentration (wt%)
0% C12
0.5% C12
1% C12
2% C12
5% C12

FIGURE 3

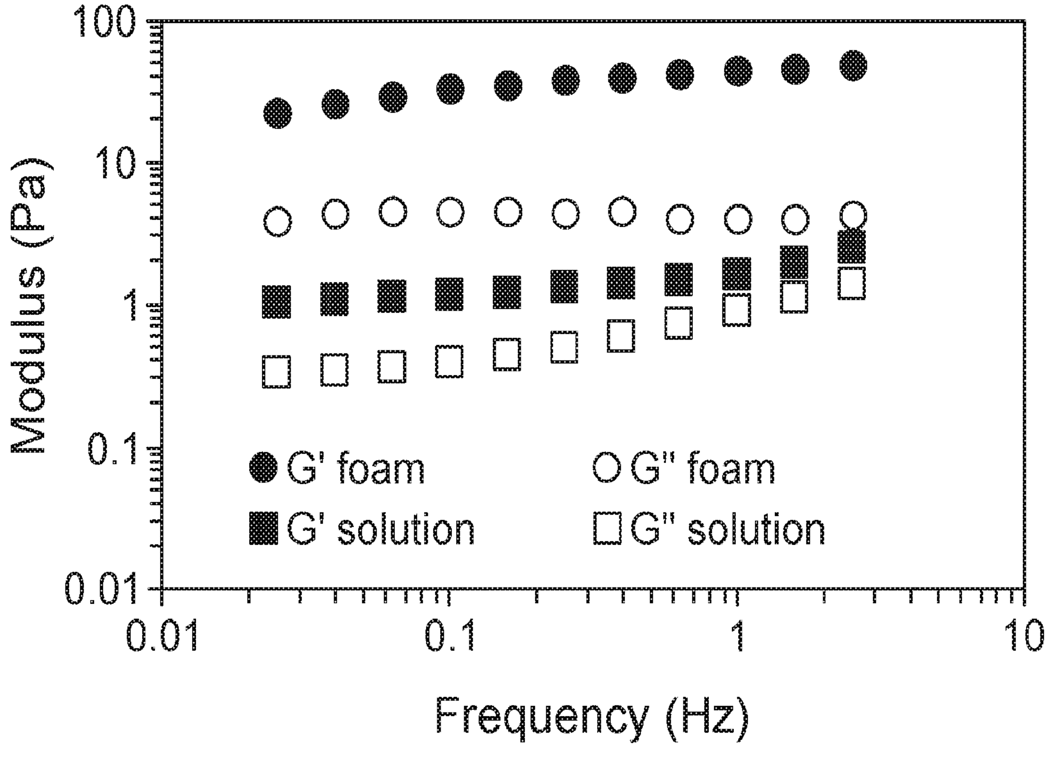
100
10
1
0.1
0.01
Modulus (Pa)
0.01
0.1
1
10
Frequency (Hz)
G' foam
G'' foam
G' solution
G'' solution

FIGURE 4

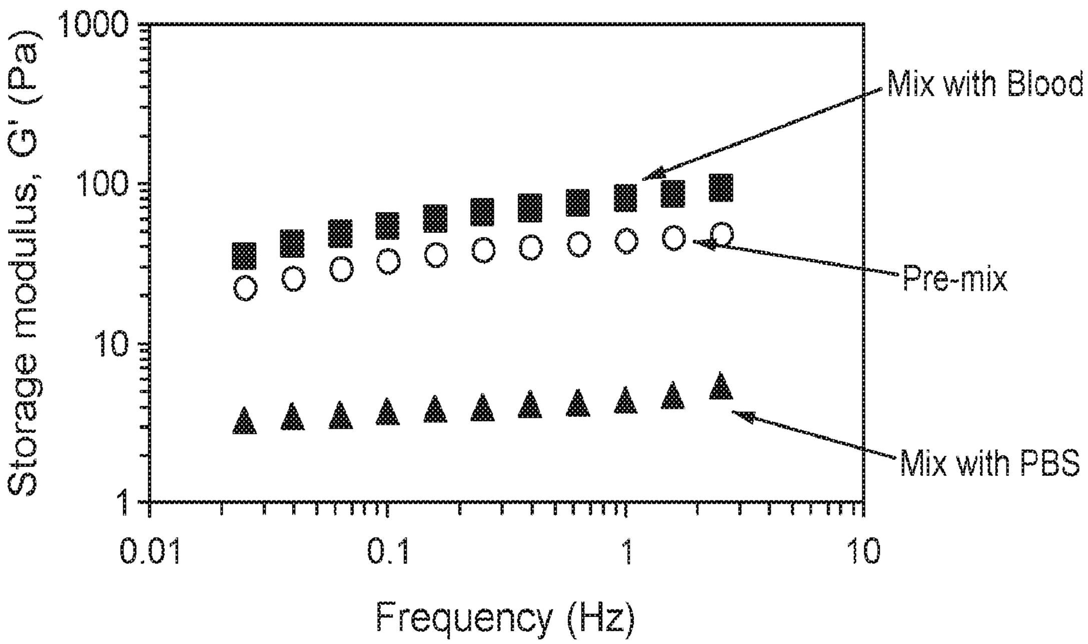
Storage modulus, G' (Pa)
1000
100
10
1
0.01
0.1
1
10
Frequency (Hz)
Mix with Blood
Pre-mix
Mix with PBS

FIGURE 5

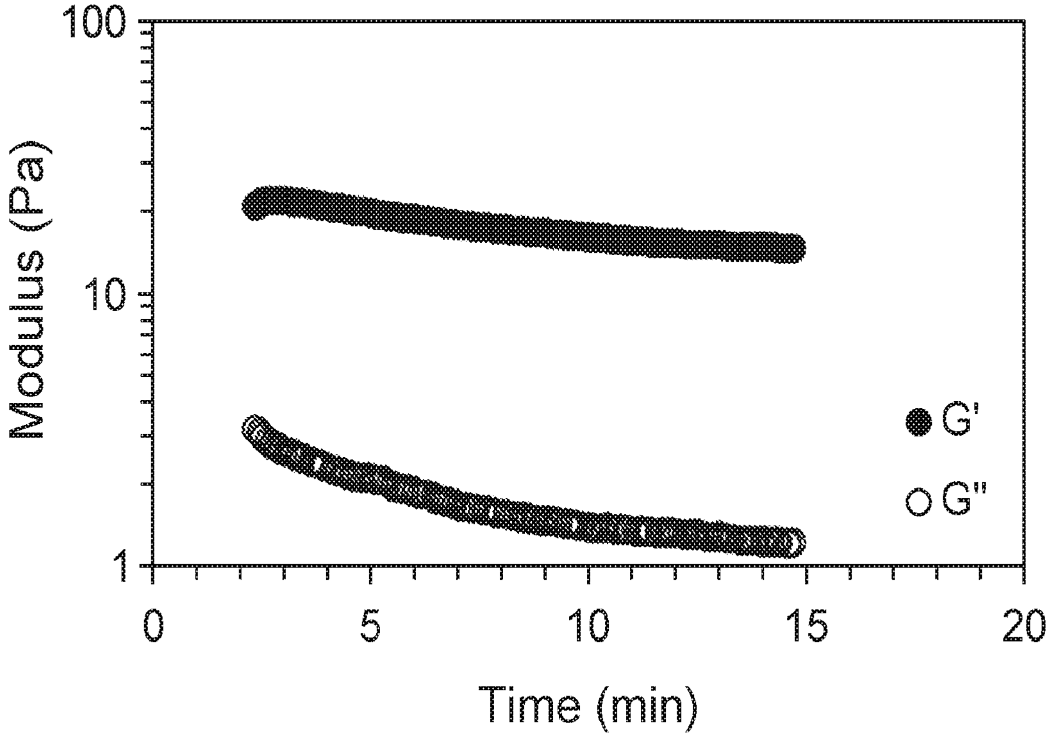

FIGURE 6

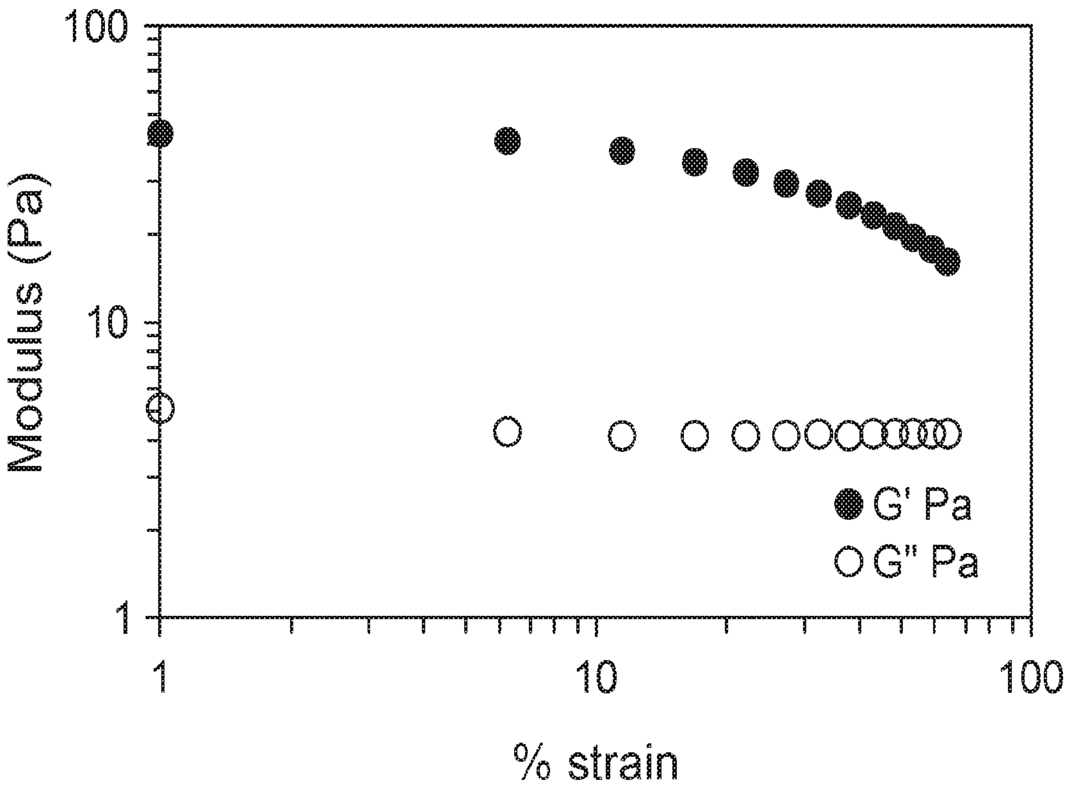

FIGURE 7

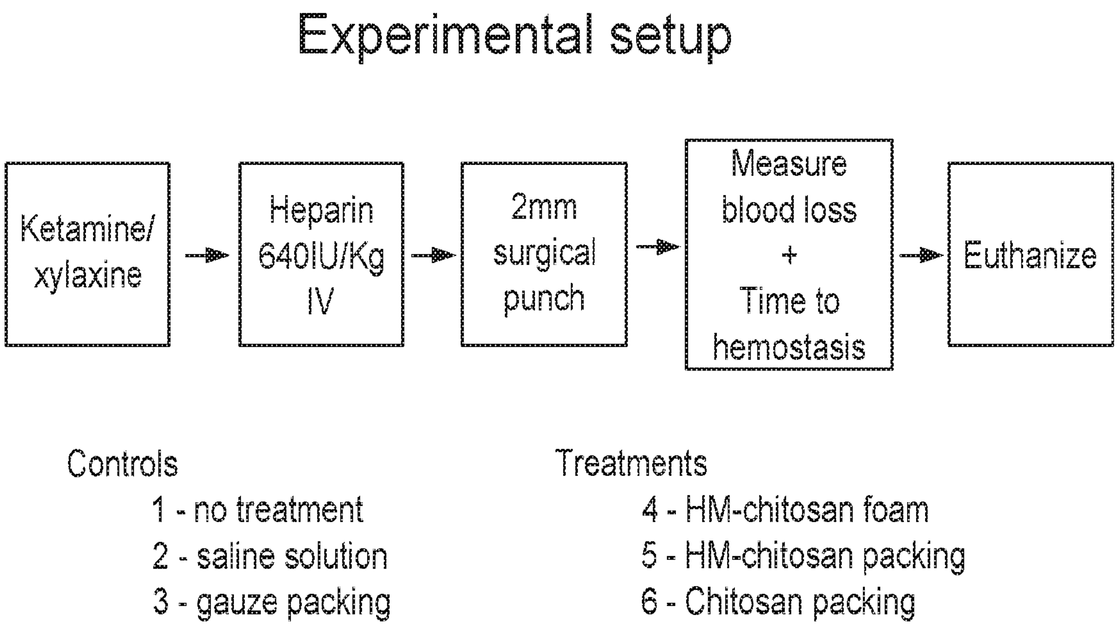
Experimental setup
Ketamine/ xylaxine
Heparin 640IU/Kg IV
2mm surgical punch
Measure blood loss + Time to hemostasis
Euthanize
Controls
1 - no treatment
2 - saline solution
3 - gauze packing
Treatments
4 - HM-chitosan foam
5 - HM-chitosan packing
6 - Chitosan packing

FIGURE 8

Experimental setup - saline/no treatment

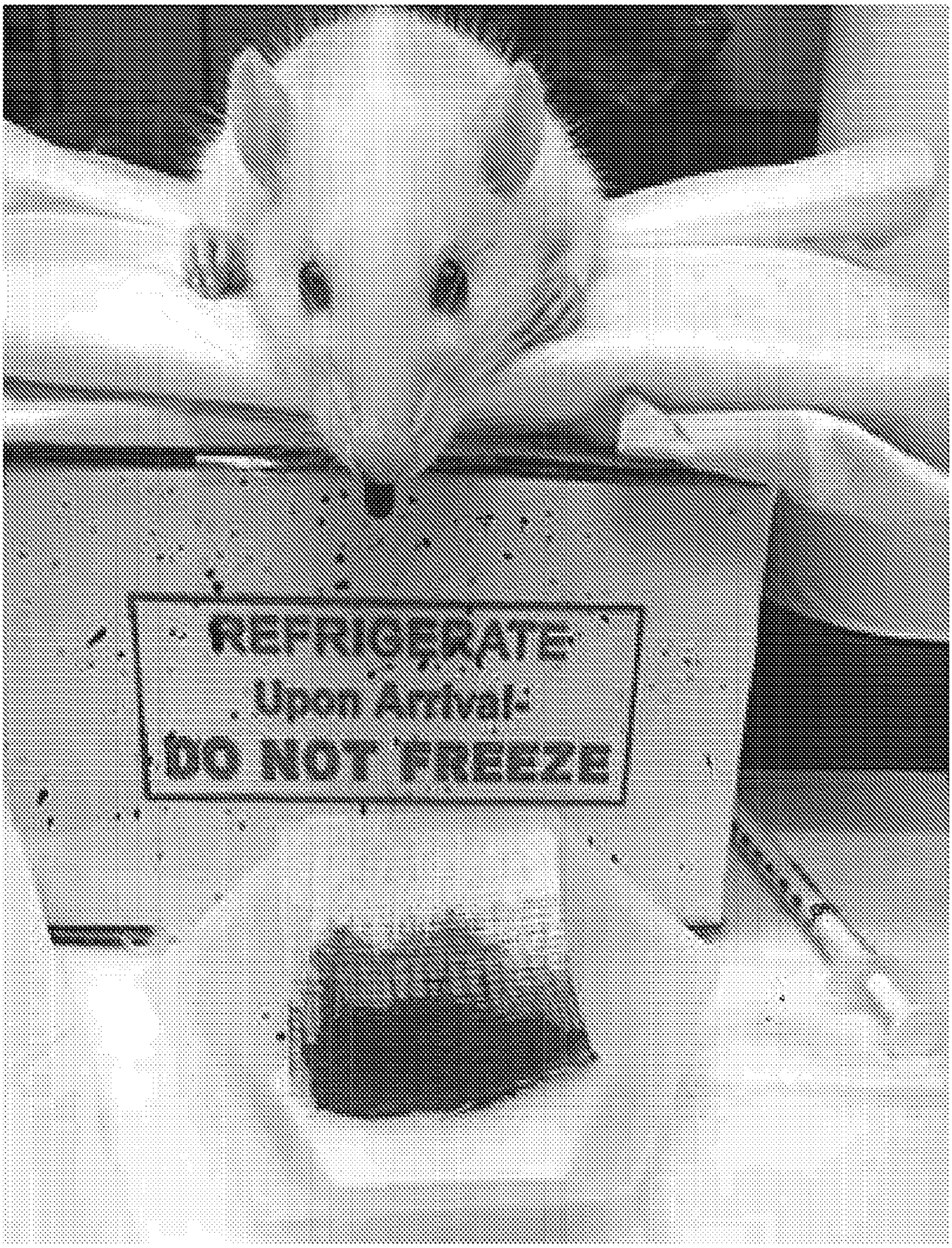

FIGURE 9

Experimental setup - HM-chitosan gauze packing

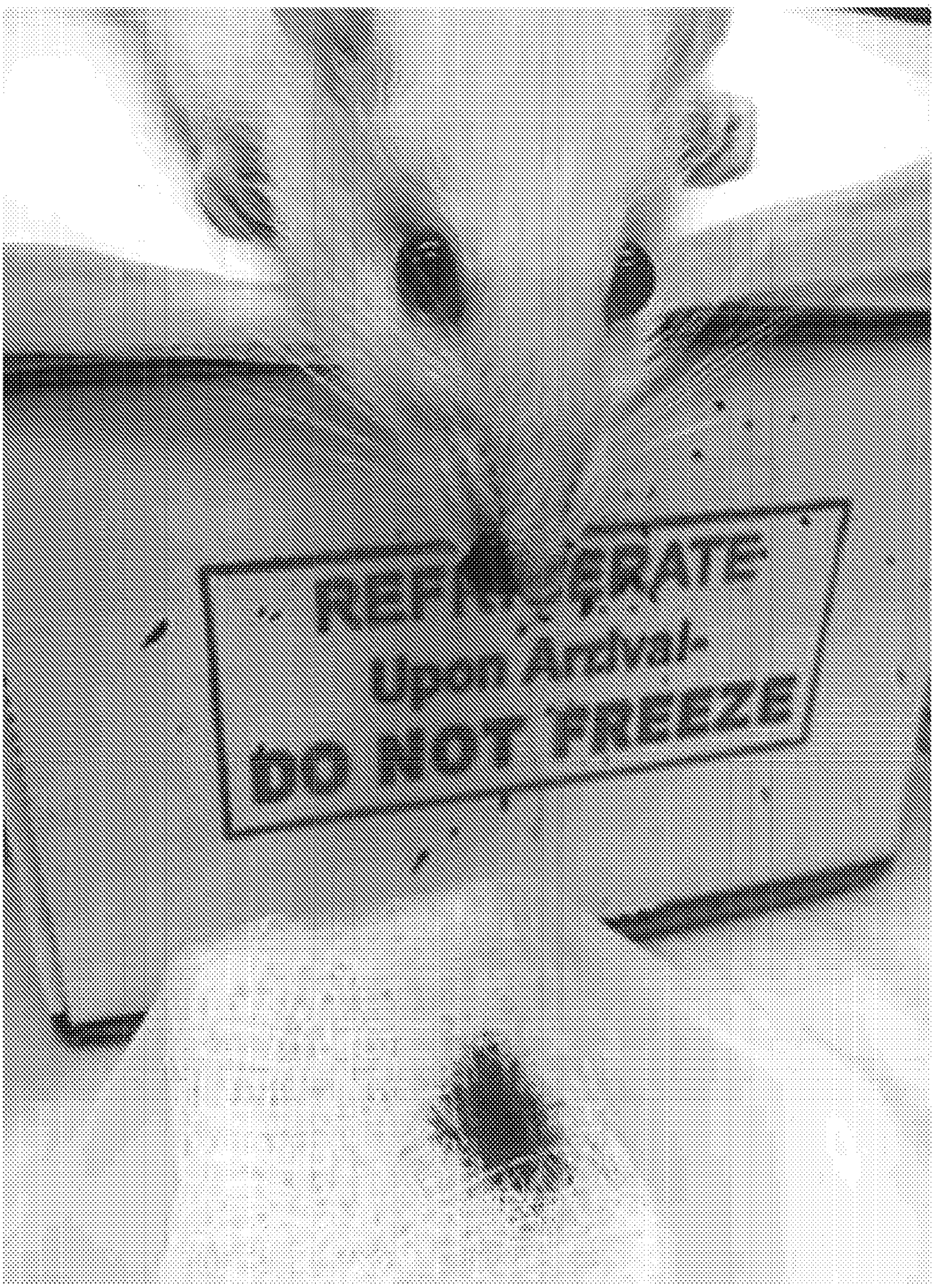

FIGURE 10

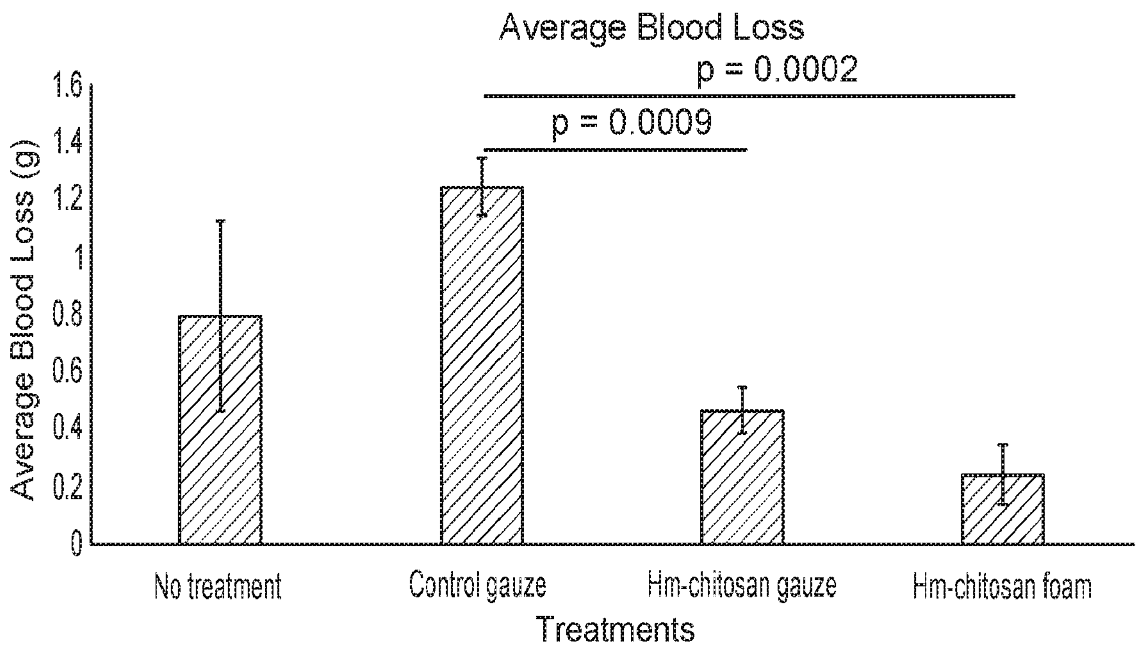


| | | | | |
|---|---|---|---|---|
| N included: | 3 | 4 | 4 | 5 |
| Total N tested: | 11 | 5 | 7 | 5 |

FIGURE 11

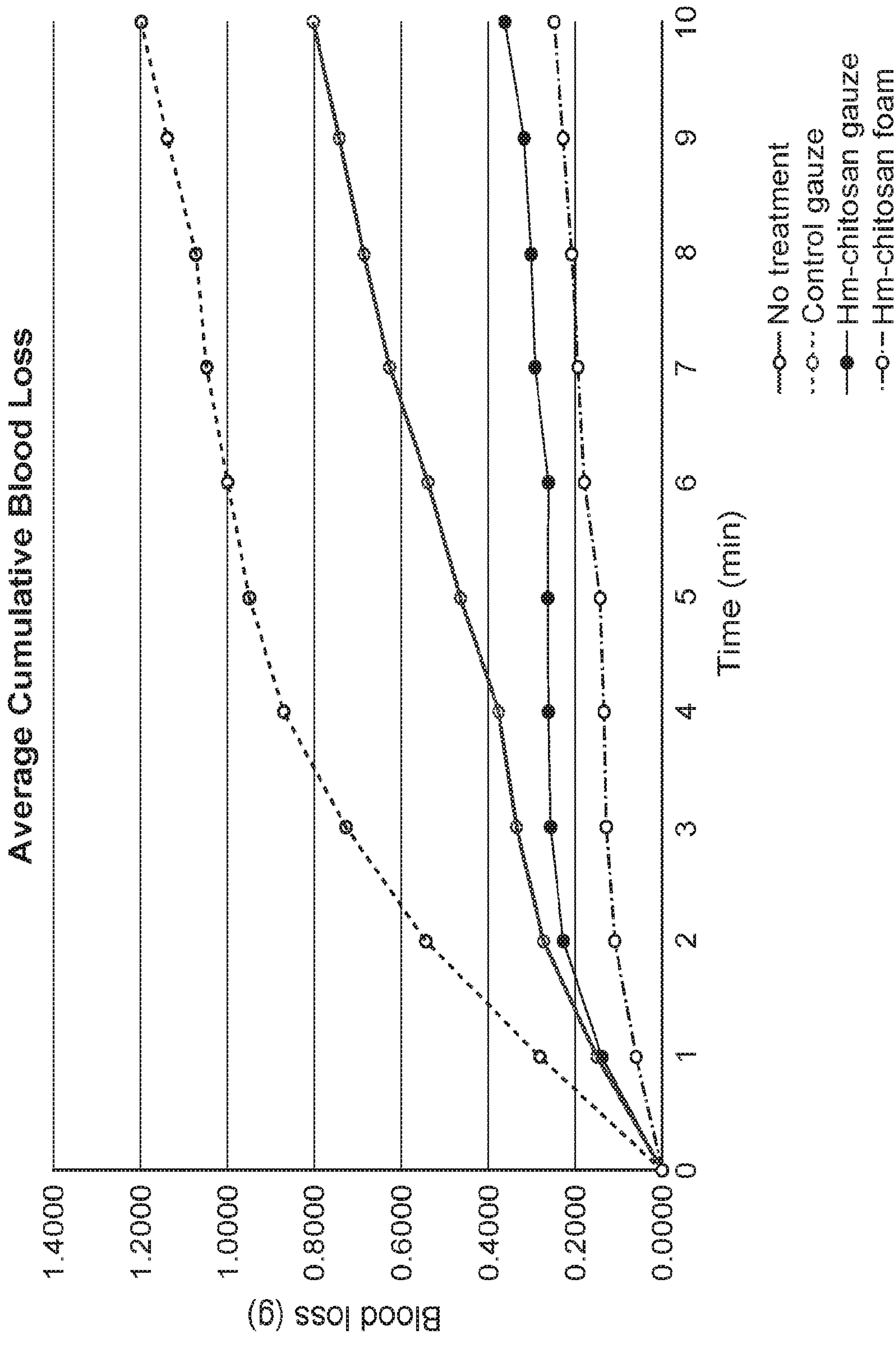
Average Cumulative Blood Loss
Blood loss (g)
0.0000
0.2000
0.4000
0.6000
0.8000
1.0000
1.2000
1.4000
Time (min)
0
1
2
3
4
5
6
7
8
9
10
No treatment
Control gauze
Hm-chitosan gauze
Hm-chitosan foam

FIGURE 12

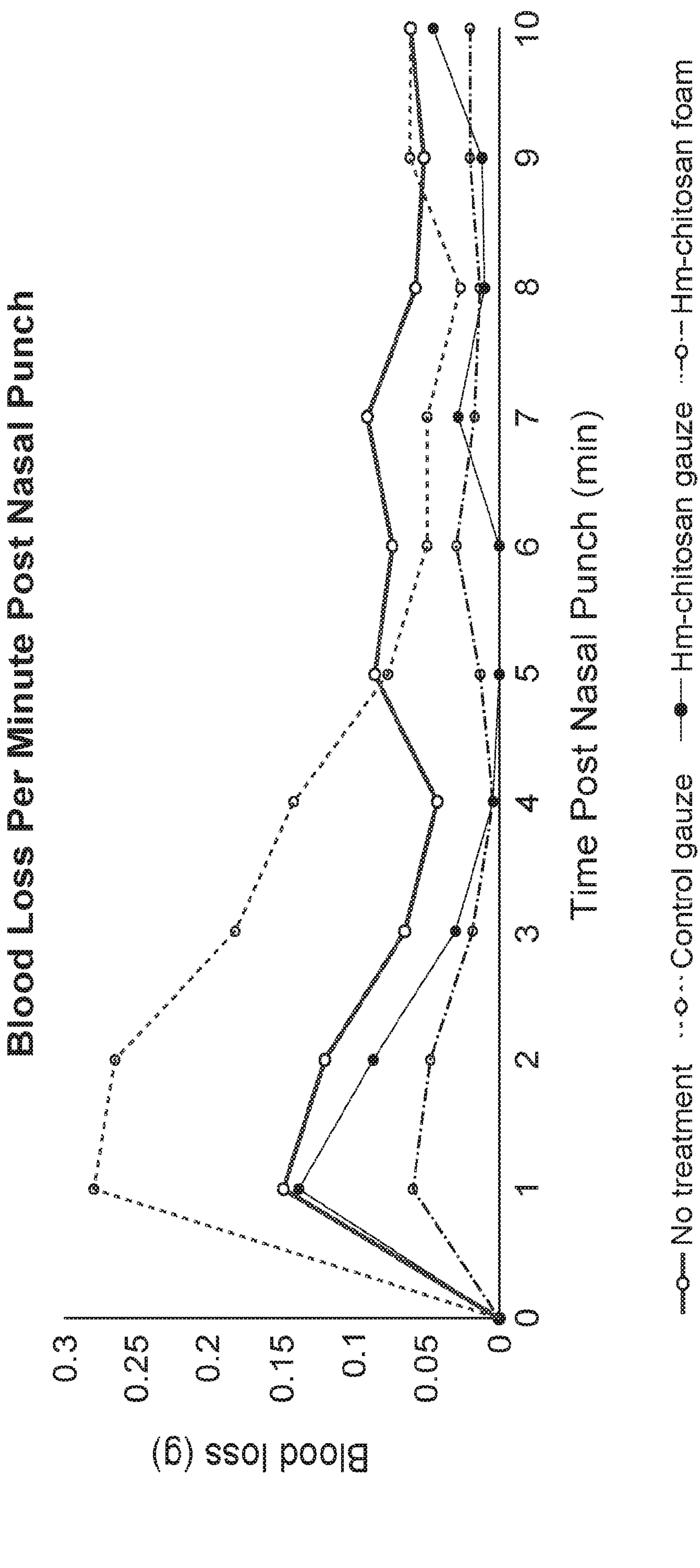
Blood Loss Per Minute Post Nasal Punch
Blood loss (g)
0
0.05
0.1
0.15
0.2
0.25
0.3
0
1
2
3
4
5
6
7
8
9
10
Time Post Nasal Punch (min)
No treatment
Control gauze
Hm-chitosan gauze
Hm-chitosan foam

FIGURE 13

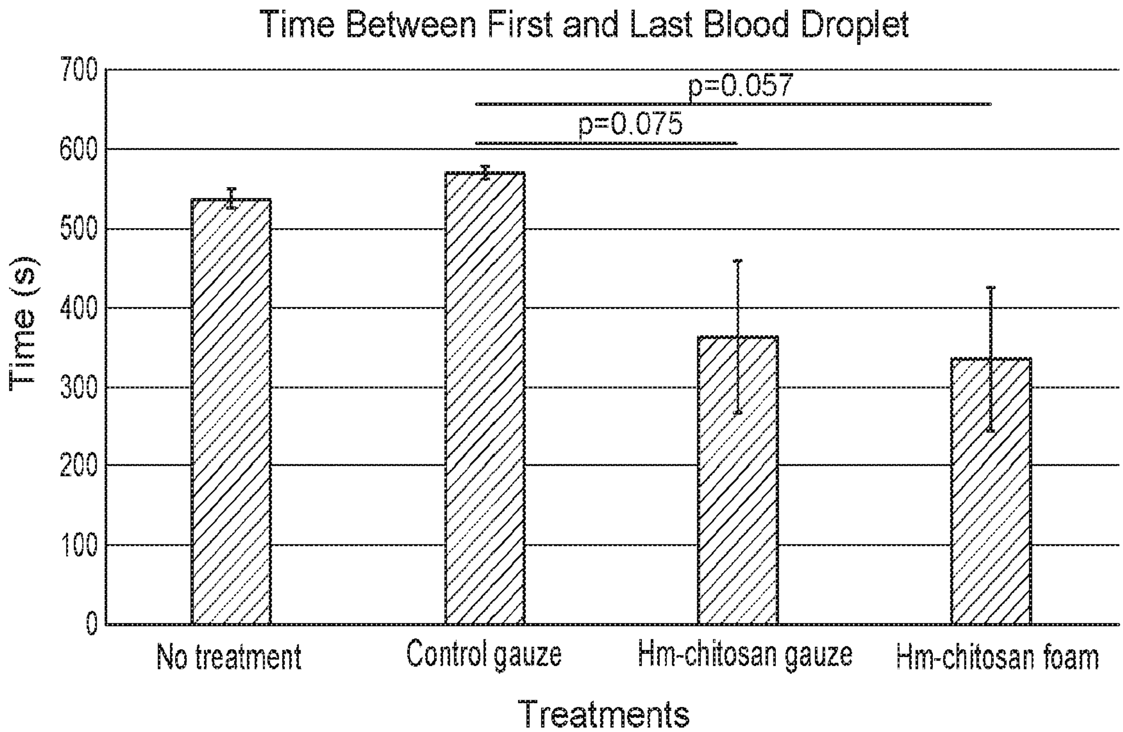
Time Between First and Last Blood Droplet
700
600
500
400
300
200
100
0
Time (s)
p=0.057
p=0.075
No treatment
Control gauze
Hm-chitosan gauze
Hm-chitosan foam
Treatments

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- US 62479903
- US 2016206777 A1